# EUROPÄISCHE PATENTANMELDUNG

(11) **EP 4 623 847 A1**
(43) Veröffentlichungstag der Anmeldung: **01.10.2025**
(21) Anmeldenummer: 25165242.6
(22) Anmeldetag: 21.03.2025
(51) Int. Cl.: A61B 18/14, A61B 18/00, A61B 18/12

(54) **VORRICHTUNG ZUR IRREVERSIBLEN ELEKTROPORATION VON GEWEBE**

(30) Priorität: 28.03.2024 DE 102024108889
(71) Anmelder: Stockert GmbH, 79111 Freiburg im Breisgau (DE)
(72) Erfinder: KEES, Fabian, 79110 Freiburg im Breisgau (DE); STORK, David, 79114 Freiburg (DE)
(74) Vertreter: Frenkel, Matthias Alexander

(57) **Zusammenfassung**

Die vorliegende Erfindung beschreibt eine Vorrichtung und ein Verfahren zur Elektroporation. Ein Ausführungsbeispiel der Vorrichtung weist einen Katheter und eine mit dem proximalen Ende des Katheters verbundene Signalgenerator-Auswerteeinheit auf.

## Beschreibung

Hier werden eine Vorrichtung und ein Verfahren zu irreversiblen Elektroporation von Gewebe vorgestellt.

In den vergangenen Jahren hat sich die Behandlung von Gewebe mittels gepulster elektrischer Felder als eine zunehmend relevante klinische Technik etabliert. Die Verwendung von kurzen Hochspannungsimpulsen und die damit verbundenen hohen elektrischen Feldstärken, die auf das Gewebe einwirken, sind jedoch bereits seit mehr als vier Jahrzehnten Gegenstand intensiver Forschung. Diese Anwendungsmethode wird als nicht-thermisches Verfahren kategorisiert, da sie auf der Abgabe kurzer Pulse mit hoher Spannungsamplitude basiert, die ein lokal starkes elektrisches Feld im Bereich von mehreren Hundert Volt pro Zentimeter zwischen aktiven Elektrodenpaaren erzeugen. Diese Feldstärke führt zur Bildung von Poren in den Zellmembranen. Überschreitet das elektrische Feld einen bestimmten Schwellenwert, der für die Bildung von Poren in den Lipiddoppelschichten der Zellmembranen erforderlich ist, und ist das Gewebe diesem Feld über einen kritischen Zeitraum ausgesetzt, wird die Elektroporation irreversibel. Die Poren bleiben permanent geöffnet, was letztendlich zum programmierten Zelltod (Apoptose) der betroffenen Zelle führt.

Die irreversible Elektroporation (IRE) ist primär ein nicht-thermisches Verfahren, welches nur eine geringfügige Erhöhung der Gewebetemperatur um einige Grad für wenige Millisekunden bewirkt. Dies grenzt es deutlich von der herkömmlichen RF-Ablation (RF: Radiofrequency) ab, bei der die Gewebetemperatur um 20°C bis 70 °C steigt und Zellen durch Hitze zerstört werden. Bei der IRE werden in der Regel bipolare Impulse eingesetzt, d.h. eine Kombination aus positiven und negativen Impulsen, um Muskelkontraktionen, die üblicherweise bei Anwendung von Gleichspannung entstehen, weitestgehend zu vermeiden. Diese Impulse können zwischen zwei bipolaren Elektroden eines Katheters oder zwischen einer Katheter-Elektrode und einer Körperoberflächenelektrode, die üblicherweise auf den Patientenrücken aufgebracht wird, angelegt werden.

Damit die IRE-Pulse die gewünschten Poren im Gewebe erzeugen, muss die durch die Pulse definierte elektrische Feldstärke E am Gewebe zwischen einem Paar aus mindestens zwei Elektroden einen gewebeabhängigen Schwellenwert Eth überschreiten. So liegt der Schwellenwert für Herzzellen beispielsweise bei etwa 500 V/cm, während er für Knochen bei 3000 V/cm liegt. Diese Unterschiede in den Schwellenfeldstärken ermöglichen eine selektive Anwendung von IRE in verschiedenem Gewebe. Um die erforderliche Feldstärke zu erreichen, hängt die an ein Elektrodenpaar anzulegende Spannung sowohl vom Zielgewebe als auch vom Abstand zwischen den Elektroden und von der Elektrodengröße selbst ab. Gleichermaßen beeinflussen diese Parameter auch den thermischen Energieeintrag während der Ablation und damit die Temperaturspitzen, die an dem zu behandelnden Gewebe auftreten können. Die angelegten Spannungen können bis zu 2000 V erreichen, was wesentlich höher ist als die typischen Spannungen von 10-200 V bei der thermischen RF-Ablation.

Der bipolare Pulsed Field Ablation-Puls (bipolare PFA-Puls) für IRE enthält einen positiven und einen negativen Puls, die zwischen zwei Elektroden mit einer Pulsbreite von 1 bis 5µs und einem Abstand zwischen den positiven und negativen Pulsen von 1 bis 5µs angelegt werden. Die bipolaren Pulse werden zu Pulsfolgen zusammengesetzt, wobei jede Folge über hundert bipolare Pulse mit einem Puls-zu-Puls-Abstand von 1 bis 10ms umfassen kann. Die Pulsfolgen bilden jeweils einen Burst, wobei sich das gesamte Pulspaket der IRE-Ablation aus 1-20 Bursts/Bursteinheiten zusammensetzt, die jeweils ein Burst-zu-Burst-Abstand von 1 bis 1000ms aufweisen. Die Gesamtdauer einer Ablation kann bis zu 10s betragen.

Das Dokument US 2022/0192741 A1 offenbart eine sphärische Struktur mit einer Vielzahl von langgestreckten Elementen, auf denen jeweils eine Vielzahl von Wandlern angeordnet sind.

Das Dokument US 2021/0169567 A1 offenbart einen Schaft eines Katheters, der dazu eingerichtet ist, in ein Organ eines Patienten eingeführt zu werden. Ein ausdehnbarer Ballon ist mit dem distalen Ende des Katheters verbunden. Auf einer Außenseite einer Membran des Ballons ist eine Vielzahl von Elektroden angeordnet.

Das Dokument WO 2022/256218 A1 offenbart einen Katheter mit einem Katheterschaft und einem Ballon. Der Ballon ist an einem distalen Endbereich des Katheterschafts angeordnet. Ein oder mehrere Elektroden sind auf einer äußeren Oberfläche, oder einer inneren Oberfläche des Ballons angeordnet.

Das Dokument CN 216495608 U offenbart einen ballonförmigen Katheter, auf dessen Außenseiten eine Vielzahl von Elektroden angeordnet sind.

Das Dokument US 2022/0022954 A1 offenbart einen Ballon-Katheter mit einer darauf angeordneten Vielzahl von Elektroden.

Das Dokument WO 2019/181634 A1 offenbart einen Ballon-Katheter mit einer Vielzahl von Elektroden, die um den Ballon angeordnet sind.

Das Dokument WO 2021/116774 A1 offenbart einen Ballon-Katheter mit einer auf einem distalen Ende des Ballon-Katheter angeordneten Vielzahl von Elektroden.

Das Dokument US 2021/0153935 A1 offenbart einen Ballon-Katheter, um dessen Außenhülle eine Elektrode gewunden ist. Aus dem distalen Endstück des Ballon-Katheters tritt spiralförmig ein Schleifenkatheter hervor, auf dem Elektroden angeordnet sind.

Des Weiteren sind die Dokumente US 2022/0233236 A1, EP 3 456 278 A2 und US 2022/0241008 A1 bekannt.

Die vorliegende Erfindung adressiert das Problem der zeitreduzierten Elektroporation.

Hierfür werden eine Vorrichtung nach Anspruch 1 und ein Verfahren nach Anspruch 13 vorgeschlagen.

Gemäß einem ersten Aspekt wird eine Vorrichtung zur irreversiblen Elektroporation eines Gewebes eines Patienten vorgeschlagen. Die Vorrichtung weist einen Katheter auf. Der Katheter weist ein proximales Ende und eine (z. B. einzige) an einem, insbesondere äußersten, distalen Ende des Katheters angeordnete, insbesondere separate/einzelne, distale Elektrode auf (Tip-Elektrode; tip = Englisch für Spitze). Der Katheter weist eine zwischen dem distalen Ende und dem proximalen Ende angeordnete Membran auf. Die Membran ist dazu eingerichtet, einen ersten, insbesondere schlauchförmigen oder kollabierten, Formzustand und einen zweiten, insbesondere ballonförmigen oder expandierten, Formzustand, einzunehmen. Der Katheter weist eine, insbesondere von der distalen Elektrode, z. B. einer Tip-Elektrode, (räumlich) getrennte, Vielzahl von Elektroden auf. Die Vielzahl von Elektroden ist auf der Membran angeordnet. Die Vorrichtung weist eine mit dem proximalen Ende des Katheters verbundene Signalgenerator-Auswerteeinheit auf, die dazu eingerichtet ist eine, insbesondere lokale, Gewebeimpedanzbestimmung und/oder eine Ablation durchzuführen.

Die Signalgenerator-Auswerteeinheit kann eine Signalgeneratoreinheit und/oder eine Auswerteeinheit und/oder eine Steuereinheit aufweisen. Die Signalgenerator-Auswerteeinheit kann dazu eingerichtet sein, eine Gewebeimpedanzbestimmung oder eine Ablation, oder eine Gewebeimpedanzbestimmung und, beispielsweise anschließend, eine Ablation durchzuführen.

Die Vorrichtung kann weiter eine an dem proximalen Ende des Katheters angeordnete proximale Elektrode aufweisen. Zusätzlich oder alternativ kann die Vorrichtung zumindest eine zusätzliche Elektrode aufweisen, die zwischen der Membran und der distalen Elektroden angeordnet ist.

Die Signalgenerator-Auswerteeinheit kann eingerichtet sein, über eine Elektrodenkonstellation ein erstes elektrisches Signal in das Gewebe abzugeben. Die Signalgenerator-Auswerteeinheit kann eingerichtet sein, ein erstes elektrisches Signal in das Gewebe abzugeben und ein zweites elektrisches Signal über die Elektrodenkonstellation aus dem Gewebe zu empfangen. Unter der Elektrodenkonstellation kann eine paarweise Zuordnung von Elektroden verstanden werden. Die Signalgenerator-Auswerteeinheit kann eingerichtet sein, Elektrodenpaare und/oder Elektrodenkonstellationen anzusteuern und/oder zwischen Elektroden-(paaren) und/oder Elektrodenkonstellationen zu schalten.

Eine Elektrodenkonstellation kann definiert/gebildet/zugeordnet werden aus der proximalen und der distalen Elektrode als erstes Elektrodenpaar und der zusätzlichen Elektrode und der distalen Elektrode als zweites Elektrodenpaar, insbesondere in dem ersten Formzustand.

Eine Elektrodenkonstellation kann definiert/gebildet/zugeordnet werden aus der proximalen und der distalen Elektrode als erstes Elektrodenpaar, und eine der Vielzahl von Elektroden und der zusätzlichen Elektrode, insbesondere in dem zweiten Formzustand, als zweites Elektrodenpaar.

Eine Elektrodenkonstellation kann definiert/gebildet/zugeordnet werden aus zwei zueinander übernächst benachbarten Elektroden der Vielzahl von Elektroden als erstes Elektrodenpaar, der distalen Elektrode und einer, insbesondere zwischen den zwei übernächst benachbarten Elektroden gelegene, weiteren Elektrode der Vielzahl von Elektroden, insbesondere in dem zweiten Formzustand, als zweites Elektrodenpaar.

Als übernächst benachbarte Elektroden können Elektroden oder ein Elektrodenpaar verstanden werden, die zwischen sich noch einen gemeinsame direkt (unmittelbar) benachbarte Elektrode aufweisen.

In einer ersten Betriebsphase kann das erste elektrische Signal als elektrisches Stromsignal und das zweite elektrische Signal als elektrisches Spannungssignal ausgebildet sein. In der ersten Betriebsphase kann die Signalgenerator-Auswerteeinheit dazu eingerichtet sein, aus dem in das Gewebe geleiteten elektrischen Stromsignal (=dem ersten elektrischen Signal) und aus dem Gewebe empfangenen elektrischen Spannungssignal (=zweiten elektrischen Signal) zumindest eine, insbesondere lokale, Gewebeimpedanz zu bestimmen.

Die Signalgenerator-Auswerteeinheit kann dazu eingerichtet sein, mittels, insbesondere (zeitlich) nacheinander gewählten/geschalteten, zumindest zwei (anzusteuernden) Elektrodenkonstellationen/Elektrodenpaaren/Elektroden, zumindest zwei, insbesondere lokale, Gewebeimpedanzen zu bestimmen.

Die Signalgenerator-Auswerteeinheit kann dazu eingerichtet sein, innerhalb einer Elektrodenkonstellation zumindest, insbesondere zeitlich nacheinander, ein anderes erstes und/oder zweites Elektrodenpaar anzusteuern und für die Elektrodenkonstellation zumindest zwei, insbesondere lokale, Gewebeimpedanzen zu bestimmen. Mit anderen Worten kann die Signalgenerator-Auswerteeinheit dazu eingerichtet sein, innerhalb einer gewählten Elektrodenkonstellation andere Elektroden innerhalb des ersten und/oder zweiten Elektrodenpaars anzusteuern, womit ein neues erstes und/oder neues zweites Elektrodenpaar gebildet wird, mit dem, insbesondere aufgrund eines anderen Signalverlaufs durch das Gewebe, eine, insbesondere lokale, Gewebeimpedanz bestimmt werden kann.

Die Signalgenerator-Auswerteeinheit kann dazu eingerichtet sein, für (eine Elektrodenkonstellation) die proximale und distale Elektrode als erstes Elektrodenpaar und eine der Vielzahl von Elektroden und die zusätzliche Elektrode, insbesondere in dem zweiten Formzustand, als zweites Elektrodenpaar, das zweite Elektrodenpaar zumindest einmalig aus zumindest einer weiteren Elektrode der Vielzahl von Elektroden und der zusätzlichen Elektroden, insbesondere in dem zweiten Formzustand, anzusteuern und zumindest eine weitere, insbesondere lokale, Gewebeimpedanz zu bestimmen.

Mit anderen Worten kann für die eine Elektrodenkonstellation das erste Elektrodenpaar aus proximaler und distaler Elektrode gebildet werden und das zweite Elektrodenpaar kann aus einer der Vielzahl von Elektroden und der zusätzlichen Elektrode gebildet werden. Die Signalgenerator-Auswerteeinheit kann dazu eingerichtet sein, nach jeder Messung des zweiten elektrischen Signals/elektrischen Spannungssignals eine weitere Elektrode der Vielzahl von Elektroden und der zusätzlichen Elektrode anzusteuern, bis über alle möglichen Elektrodenkombinationen der Vielzahl von Elektroden und der zusätzlichen Elektrode jeweils ein zweites elektrisches Signal/elektrisches Spannungssignal gemessen wurden.

Die Signalgenerator-Auswerteeinheit kann dazu eingerichtet sein, für (eine Elektrodenkonstellation) zwei zueinander übernächst benachbarte Elektroden der Vielzahl von Elektroden als erstes Elektrodenpaar und die distale Elektrode und eine, insbesondere zwischen den zwei übernächst benachbarten Elektroden gelegene, weitere Elektrode der Vielzahl von Elektroden, insbesondere in dem zweiten Formzustand, als zweites Elektrodenpaar zu bilden. Die Signalgenerator-Auswerteeinheit kann dazu eingerichtet sein, das erste Elektrodenpaar zumindest einmalig aus zwei weiteren übernächst benachbarten Elektroden der Vielzahl von Elektroden, insbesondere in dem zweiten Formzustand, und das zweite Elektrodenpaar aus der distalen Elektrode und eine, insbesondere zwischen den zwei weiteren übernächst benachbarten Elektroden gelegene, weitere Elektrode der Vielzahl von Elektroden, insbesondere in dem zweiten Formzustand, anzusteuern/zu bilden und zumindest eine weitere, insbesondere lokale, Gewebeimpedanz zu bestimmen.

In einer zweiten Betriebsphase kann das elektrische Signal als elektrisches Spannungssignal ausgebildet sein. Die Signalgenerator-Auswerteeinheit kann dazu eingerichtet sein, das elektrische Spannungssignal gemäß eines zu wählenden Burst-Signal-Protokolls zu generieren und über ein erstes Elektrodenpaar in das Gewebe zu leiten. Die Signalgenerator-Auswerteeinheit kann, insbesondere in der zweiten Betriebsphase, ein Elektrodenpaar ansteuern.

Das erste Elektrodenpaar kann, insbesondere in einem zweiten Formzustand, aus der distalen Elektrode und einer der Vielzahl von Elektroden gebildet sein. Das erste Elektrodenpaar kann, insbesondere in einem zweiten Formzustand, aus zwei, insbesondere (unmittelbar) benachbarten, Elektroden der Vielzahl von Elektroden, gebildet sein.

Die Vorrichtung kann eine mit der Signalgenerator-Auswerteeinheit verbundene Gegenelektrode, insbesondere Körperflächen-Gegenelektrode, aufweisen. Das erste Elektrodenpaar kann aus einer der Vielzahl von Elektroden und der Gegenelektrode, insbesondere in dem zweiten Formzustand, gebildet sein. Das erste Elektrodenpaar kann, insbesondere in dem ersten Formzustand, aus der distalen Elektrode und der Gegenelektrode gebildet sein.

In einer ersten Betriebsphase kann das erste elektrische Signal als elektrisches Stromsignal und das zweite elektrische Signal als elektrisches Spannungssignal ausgebildet sein. In einer ersten Betriebsphase kann die Signalgenerator-Auswerteeinheit dazu eingerichtet sein, aus dem in das Gewebe geleiteten elektrischen Stromsignal und dem aus dem Gewebe empfangenen elektrischen Spannungssignal zumindest eine, insbesondere globale und/oder lokale, Gewebeimpedanz zu bestimmen. Das erste elektrische Signal kann über ein erstes Elektrodenpaar in das Gewebe geleitet werden. Das zweite elektrische Signal kann über das erste Elektrodenpaar empfangen werden. Anders ausgedrückt kann über das gleiche oder dasselbe Elektrodenpaar ein elektrischer Strom in das Gewebe geleitet und eine elektrische Spannung empfangen werden.

Die Signalgenerator-Auswerteeinheit kann dazu eingerichtet sein, innerhalb eines Elektrodenpaars oder ausgehend von einem Elektrodenpaar, insbesondere zeitlich nacheinander, zu zumindest einer weiteren Elektrode der Vielzahl von Elektroden zu schalten. Anders ausgedrückt, eine Elektrode des Elektrodenpaars kann durch eine weitere der Vielzahl von Elektroden ersetzt werden. Auf diese Weise kann ein neues Elektrodenpaar gebildet werden. Dies kann, insbesondere zeitlich nacheinander, wiederholt durchgeführt werden.

Die Elektrode(n) (insbesondere die Vielzahl von Elektroden, die proximale, die distale und/oder die zusätzliche Elektrode) kann/können mittels untereinander und zur unmittelbaren Umgebung isolierten, insbesondere außen oder innen auf/in dem Katheter angeordneten, elektrischen Leitung(en) über das proximalen Ende des Katheters mit der Signalgenerator-Auswerteeinheit verbunden sein.

Die Signalgenerator-Auswerteeinheit kann dazu eingerichtet sein, insbesondere in einer ersten und/oder zweiten Betriebsphase, ein erstes elektrisches Signal auf Basis eines Burst-Signal-Protokolls zu generieren und an ein erstes Elektrodenpaar zu leiten/senden.

Formate des Burst-Signalfolge-Protokolls können Eigenschaften und den Energiebetrag pro Burst vorgeben. Das/Die Format/e kann/können aufweisen: eine erste Anzahl an Bursts innerhalb der Burst-Signalfolge, zumindest einen ersten zeitlichen Abstand zwischen zumindest zwei aufeinanderfolgenden Bursts der Burst-Signalfolge, eine zweite Anzahl von bipolaren Pulsen innerhalb eines Bursts, zumindest einen zweiten zeitlichen Abstand zwischen zumindest zwei aufeinanderfolgenden bipolaren Pulsen innerhalb eines Bursts, einen dritten zeitlichen Abstand zwischen einem positiven und negativen Puls zumindest eines bipolaren Pulses, eine Pulsbreite eines positiven und/oder eines negativen Pulses zumindest eines bipolaren Pulses, und/oder einen Wert einer Pulsauslenkung eines positiven und/oder eines negativen Pulses zumindest eines bipolaren Pulses.

Eine erste Anzahl an Bursts innerhalb der Burst-Signalfolge kann in einem Wertebereich von 1 bis 100 Bursteinheiten liegen.

Zumindest ein erster zeitlicher Abstand zwischen zwei aufeinanderfolgenden Bursts der Burst-Signalfolge kann in einem Wertebereich von 1ms bis 1000ms liegen. Eine zweite Anzahl von bipolaren Pulsen innerhalb eines Bursts kann in einem Wertebereich von 1 bis 300 bipolaren Pulseinheiten liegen.

Zumindest ein zweiter zeitlicher Abstand zwischen zumindest zwei aufeinanderfolgenden bipolaren Pulsen innerhalb eines Bursts kann in einem Wertebereich von 1 bis 10ms liegen. Ein dritter zeitlicher Abstand zwischen einem positiven und negativen Puls kann in einem Wertebereich zwischen 1 bis 5µs liegen.

Eine Pulsbreite eines positiven und/oder eines negativen Pulses kann in einem Wertebereich zwischen 1 bis 10µs liegen. Eine Pulsbreite eines positiven Pulses kann sich von einer Pulsbreite eines negativen Pulses unterscheiden.

Ein Wert einer Pulsauslenkung eines positiven Pulses kann in einem Wertebereich von 200 bis 2000V liegen. Ein Wert einer Pulsauslenkung eines negativen Pulses kann in einem Wertebereich von -200 bis -2000V liegen.

Gemäß einem zweiten Aspekt wird ein Verfahren zur irreversiblen Elektroporation eines Gewebes eines Patienten vorgeschlagen. Das Verfahren umfasst ein Bereitstellen eines Katheters. Der Katheter weist ein proximales Ende und eine an einem distalen Ende des Katheters angeordnete distale Elektrode auf. Der Katheter weist eine zwischen dem distalen Ende und dem proximalen Ende angeordnete Membran auf. Die Membran ist dazu eingerichtet, einen ersten oder zweiten Formzustand einzunehmen. Der Katheter weist eine Vielzahl von Elektroden auf, die auf der Membran angeordnet sind. Das Verfahren umfasst ein Bereitstellen einer mit dem proximalen Ende des Katheters verbundenen Signalgenerator-Auswerteeinheit. Das Verfahren umfasst ein Durchführen einer Gewebeimpedanzbestimmung und/oder ein Durchführen einer Ablation. Anders ausgedrückt, das Verfahren umfasst (i) ein Durchführen einer Gewebeimpedanzbestimmung oder (ii) ein Durchführen einer Ablation oder (iii) ein Durchführen einer Gewebeimpedanzbestimmung und ein Durchführen einer Ablation, beispielsweise ein an die Gewebeimpedanzbestimmung anschließendes oder nachfolgendes Durchführen einer Ablation.

Weitere Merkmale, Eigenschaften, Vorteile und mögliche Abwandlungen werden für einen Fachmann anhand der nachstehenden Beschreibungen deutlich, in der auf die beigefügten Zeichnungen Bezug genommen ist.
Figur 1 ist eine schematische Darstellung eines bipolaren IRE-Impulses entsprechend einer beispielhaften Ausführung der Erfindung.
Figur 2 ist eine schematische Darstellung eines Puls Protokolls mit mehreren Serien bzw. Bursts bipolarer Impulse entsprechend einer beispielhaften Ausführung der Erfindung.
Figur 3 zeigt schematische Darstellungen einer Vorrichtung mit expandierter Ballon-Membran.
Figur 4 zeigt schematische Darstellungen der Vorrichtung aus Figur 3 mit eingefalteter Membran in der fokalen-Konfiguration.
Figur 5 zeigt schematische Verschaltungen der Elektroden zur lokalen Impedanzmessung für die fokale Katheter-Konfiguration.
Figur 6 zeigt schematische Verschaltungen der Elektroden zur lokalen Impedanzmessung für die "One-Shot" Katheter-Konfiguration.
Figur 7 zeigt schematische weitere Verschaltungen der Elektroden zur lokalen Impedanzmessung für die "One-Shot" Katheter-Konfiguration.
Figur 8 zeigt schematisch die möglichen Ablations-Modi der Vorrichtung im expandierte Formzustand der Membran.
Figur 9 zeigt schematisch die möglichen Ablations-Modi der Vorrichtung für den kollabierten Formzustand der Membran.
Figur 10 zeigt schematisch die Messung einer globalen Gewebeimpedanz der Vorrichtung in einem expandierten Zustand der Membran.
Figur 11 zeigt schematisch die Messung einer globalen Gewebeimpedanz der Vorrichtung in einem kollabierten Zustand der Membran.

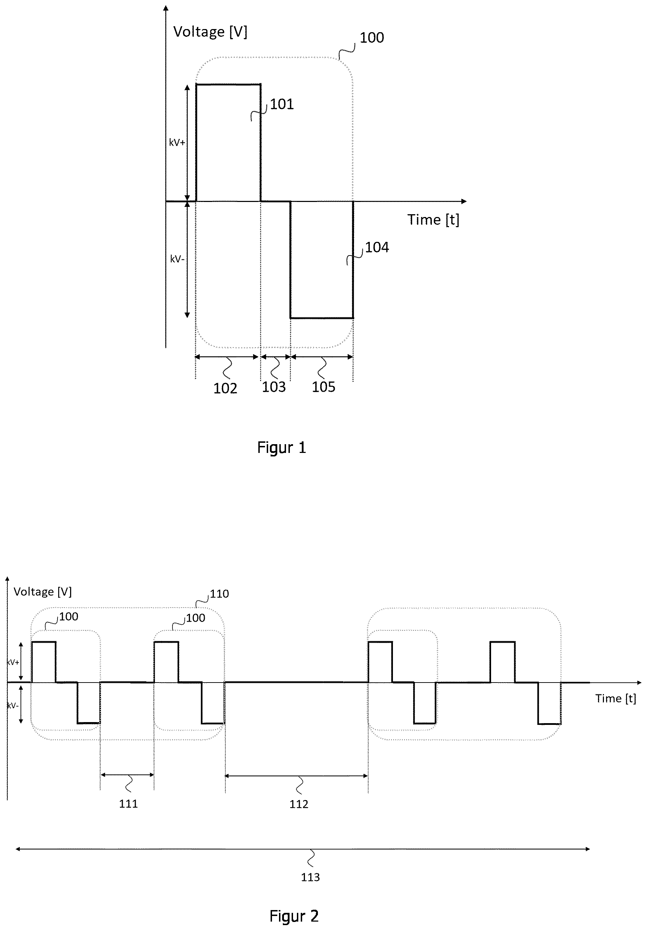

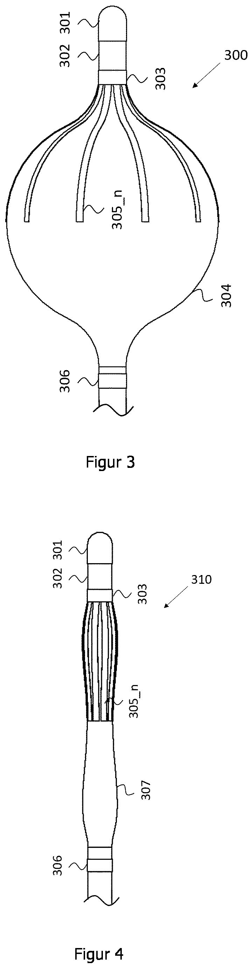

Figur 1 zeigt eine schematische Darstellung eines bipolaren Pulses 100, der von der Signalgenerator-Auswerteeinheit generiert wird, wenn die Signalgenerator-Auswerteeinheit gemäß eines Burst-Signalfolge-Protokoll ein erstes elektrisches Signal in der ersten Betriebsphase einer Vorrichtung generiert. Im vorliegenden Beispiel sind die Formate, die die Eigenschaften des bipolaren Pulses 100 bestimmen, durch einen Nutzer vordefiniert worden. Die Werte der Pulsauslenkung kV+, kV- des positiven 101 und negativen Pulses 104 sind im gezeigten Beispiel ±500kV. Der dritte zeitliche Abstand 103 zwischen dem positiven 101 und dem negativen Puls 104 ist 2,5µs. Die Pulsbreite 102 des positiven Pulses 101 unterscheidet sich von der Pulsbreite 105 des negativen Pulses 104. Der Unterschied der Pulsbreiten ist in der Figur 1 nicht dargestellt. Zur Erläuterung der Gewebeimpedanzmessung mit der/den von der Signalgenerator-Auswerteeinheit angesteuerten Elektrodenkonstellation(en) wird auf die Figuren 4 bis 9 verwiesen.

Figur 2 zeigt schematisch ein erstes elektrisches Signal in einer zweiten Betriebsphase der Vorrichtung. Das erste elektrische Signal ist als Burst-Signalfolge ausgebildet. Zu erkennen sind zwei Bursts, von denen einer mit dem Bezugszeichen 110 versehen ist. Jeder Burst weist zwei bipolare Pulse 100 auf. Jeder in der Burst-Signalfolge vorkommende bipolare Puls 100 weist die Eigenschaften des Formats aus der vorhergehenden Figurenbeschreibung auf. Die erste Anzahl an Bursts, hier exemplarisch zwei Bursts, der zweite zeitlicher Abstand 111, und der erste zeitliche Abstand 112 zwischen zwei aufeinanderfolgenden Bursts 110, sind von einem Nutzer vor einer ersten Betriebsphase festgelegt worden. Die zu erkennende Burst-Signalfolge erstreckt sich über eine Dauer 113, die der Dauer der irreversiblen Elektroporation entspricht.

Figur 3 zeigt schematische Darstellungen der Vorrichtung, in der die Membran 304 expandiert ist. Die Vorrichtung ist in einer "One-Shot"-Konfiguration 300. Die Vorrichtung weist einen Schaft 302 auf, welcher in steuerbarer oder nicht-steuerbarer Konfiguration vorliegen kann, sowie eine darauf dauerhaft befestigte, befüllte Membran 304. Steuerbar bezieht sich z. B. auf die Möglichkeit, mit einem am Schaft angeordneten Griff der Vorrichtung durch eine Drehbewegung des Griffs eine Biegung zu verleihen und die Vorrichtung im Organ eines Patienten zu steuern. Auf dem Schaft 302 ist eine distale Elektrode 301, sowie eine zusätzliche Elektrode 303 angeordnet. Die zusätzliche Elektrode 303 ist distal zur Membran 304 angeordnet. Eine proximale Elektrode 306 ist proximal von der Membran 304 angeordnet. Die Elektroden 301, 303 und 306 sind über eine oder mehrere elektrisch zueinander isolierte Leitungen, die sich vom proximalen Ende über den Schaft hin bis zu den Elektroden erstrecken, elektrisch kontaktiert. Auf einer Außenfläche der Membran 304 ist eine Vielzahl von Elektroden 305_n angeordnet, wobei n hier der Anzahl an Elektroden entspricht und dies, beispielhaft und ohne hierauf beschränkt zu sein, bis zu 15 Elektroden sein können. Diese werden über eine oder mehrere Leiterbahnen, die sich von dem proximalen Ende der Vorrichtung über den Schaft hin bis zu den Elektroden 305_n erstrecken, elektrisch verbunden. Die eine oder mehrere der Leiterbahnen sind derart abgedeckt, dass sie sowohl untereinander als auch zur äußeren Umgebung elektrisch isoliert sind.

Figur 4 zeigt schematische Darstellungen der Vorrichtung, in der die Membran 304 kollabiert/zusammengefaltet ist. Die Vorrichtung ist in einer fokalen Konfiguration 310. Die Vorrichtung weist einen Schaft 302 auf, welcher in steuerbarer oder nicht-steuerbarer Konfiguration vorliegen kann, sowie eine darauf dauerhaft befestigte, Membran 307 in kollabierter Form. Auf dem Schaft 302 ist eine distale Elektrode 301 sowie eine zusätzliche Elektrode 303 angeordnet. Die zusätzliche Elektrode 303 ist distal zu der Membran 304 angeordnet. Eine proximale Elektrode 306 ist proximal von der Membran 304 angeordnet. Die Elektroden 301, 303 und 306 sind elektrisch kontaktiert, wobei diese Drähte über elektrische Leitungen zu dem proximalen Ende der Vorrichtung geführt werden und elektrisch zueinander isoliert sind. Auf einer Außenfläche der Membran 307 ist eine Vielzahl von Elektroden 305_n angeordnet, wobei n hier der Anzahl an Elektroden entspricht und dies, beispielhaft und ohne hierauf beschränkt zu sein, bis zu 15 Elektroden sein können. Diese werden über eine oder mehrere Leiterbahnen, die sich von dem proximalen Ende der Vorrichtung über den Schaft hin bis zu den Elektroden erstrecken, elektrisch verbunden. Die eine oder mehrere der Leiterbahnen sind derart abgedeckt, dass sie sowohl untereinander als auch zur äußeren Umgebung elektrisch isoliert sind.

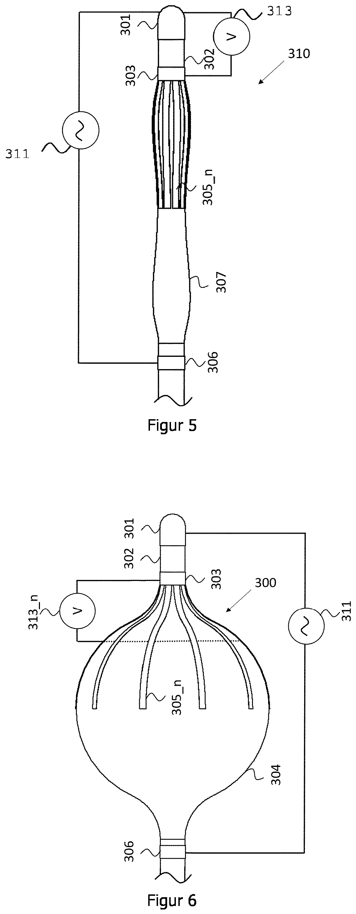

Figur 5 zeigt die Vorrichtung in der fokalen Konfiguration 310 zur Ansteuerung der Elektroden und zur Bestimmung der lokalen Impedanz des Gewebes. Die Vorrichtung weist eine distale Elektrode 301, eine zusätzliche Elektrode 303 und eine proximale Elektrode 306 auf. Die zwischen der zusätzlichen Elektrode 303 und der proximalen Elektrode 306 angeordnete Membran 307 ist in einem ersten Formzustand; die Membran ist kollabiert. Auf der Membran 307 ist eine Vielzahl von Elektroden 305_n angeordnet. Hierbei ist das erste elektrische Signal als elektrisches Stromsignal 313 ausgebildet. Das zweite elektrische Signal ist als elektrisches Spannungssignal ausgebildet. Die hier nicht dargestellte Signalgenerator-Auswerteeinheit steuert die entsprechende Elektrodenkonstellation mit den entsprechenden Elektrodenpaaren an.

Das elektrische Signal 311 wird zwischen der distalen Elektrode 301, auch Spitzen-Elektrode genannt, und der proximalen Elektrode 306 angelegt und bildet das erste Elektrodenpaar, über welches der elektrische Strom in das Gewebe geleitet wird, welches mit dem Elektrodenpaar in Kontakt steht. Über ein zweites Elektrodenpaar, hier die distale Elektrode 301 und die zusätzliche Elektrode 303 wird ein elektrisches Spannungssignal 315 gemessen. Das erste aus den Elektroden 301, 306 gebildete Elektrodenpaar und das zweite aus den Elektroden 301, 303 gebildete Elektrodenpaar bilden zusammen eine Elektrodenkonstellation. Über den Zusammenhang des Ohm'schen Gesetztes kann anhand des über das erste Elektrodenpaar eingeleiteten elektrischen Stroms und der über das zweite Elektrodenpaar empfangenen elektrischen Spannung die lokale Gewebeimpedanz ermittelt werden.

Figur 6 zeigt die Vorrichtung in der "One-Shot"-Konfiguration 300 zur Ansteuerung der Elektroden und zur Bestimmung der lokalen Gewebeimpedanz des Gewebes. Die Vorrichtung weist eine distale Elektrode 301, eine zusätzliche Elektrode 303 und eine proximale Elektrode 306 auf. Die zwischen der zusätzlichen Elektrode 303 und der proximalen Elektrode 306 angeordnete Membran 304 ist in einem zweiten Formzustand; die Membran 304 ist expandiert. Auf der Membran 204 ist eine Vielzahl von Elektroden 305_n angeordnet. Hierbei ist das erste elektrische Signal als elektrisches Stromsignal 311 ausgebildet. Das zweite elektrische Signal ist als elektrisches Spannungssignal 311 ausgebildet. Die hier nicht dargestellte Signalgenerator-Auswerteeinheit steuert die entsprechende Elektrodenkonstellation mit den entsprechenden Elektrodenpaaren an, um über die Elektrodenkonstellation Strom in das Gewebe zu leiten und Spannung am Gewebe zu messen.

Der elektrischer Strom 311 wird zwischen der distalen Elektrode 301 und der proximalen Elektrode 306 (erstes Elektrodenpaar) angelegt. Im vorliegenden Fall ist die proximale Elektrode 306 als Ring-Elektrode ausgebildet. Zur Gewebeimpedanzbestimmung mittels Ohm'schen Gesetz werden Spannungen 313_n von jeder der Vielzahl von Elektroden 305_n zu der zusätzlichen Elektrode 303 gemessen (zweites Elektrodenpaar). Dazu steuert/schaltet die Signalgenerator-Auswerteeinheit nach erfolgter Messung zwischen einer der Vielzahl von Elektroden 305_n und der zusätzlichen Elektroden 303 auf eine weitere Elektrode der Vielzahl von Elektroden 305_n bis alle der Vielzahl von Elektroden 305_n für eine Messung der elektrischen Spannung 313_n zwischen der Vielzahl von Elektroden 305_n und der zusätzlichen Elektrode 303 angesteuert wurden. Dabei entspricht n hier erneut der Anzahl der Elektroden auf der Membran 304, wodurch sich entsprechend auch die gleiche Anzahl an ermittelten lokalen Impedanzen ergeben und die Eigenschaften des Zielgewebes sehr selektiv ermittelt werden können.

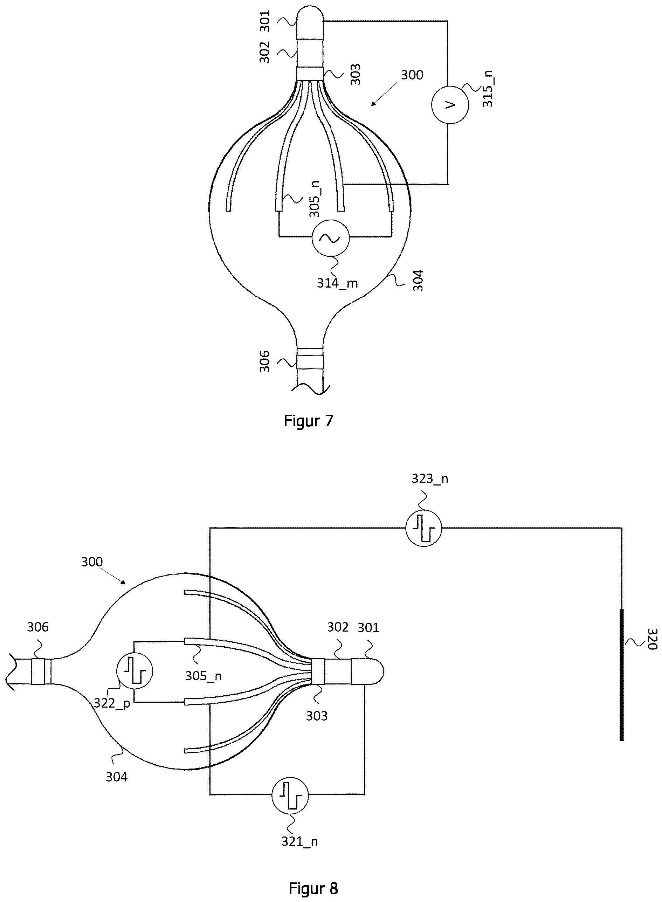

Die Figur 7 zeigt schematisch die Vorrichtung in der "One-Shot"-Konfiguration 300 zur Ansteuerung der Elektroden und zur Bestimmung der lokalen Gewebeimpedanz des Gewebes. Die Vorrichtung weist eine distale Elektrode 301, eine zusätzliche Elektrode 303 und eine proximale Elektrode 306 auf. Die zwischen der zusätzlichen Elektrode 303 und der proximalen Elektrode 306 angeordnete Membran 304 ist in einem zweiten Formzustand; die Membran 304 ist expandiert. Auf der Membran 304 ist eine Vielzahl von Elektroden 305_n angeordnet. Hierbei ist das erste elektrische Signal als elektrisches Stromsignal 314_m ausgebildet. Das zweite elektrische Signal ist als elektrisches Spannungssignal 315_n ausgebildet. Die hier nicht dargestellte Signalgenerator-Auswerteeinheit steuert die entsprechende Elektrodenkonstellation mit den entsprechenden Elektrodenpaaren an.

Der elektrische Strom 314_m wird zwischen zwei übernächst benachbarten Elektroden der Vielzahl von Elektroden 305_n angelegt (erstes Elektrodenpaar). Zur Gewebeimpedanzbestimmung mittels Ohm'schen Gesetz wird die Spannung 315_n zwischen einer Elektrode der Vielzahl von Elektroden und der distalen Elektrode 301 gemessen (zweites Elektrodenpaar). Die eine Elektrode der Vielzahl von Elektroden des zweiten Elektrodenpaars ist jene, die sich die übernächst benachbarten Elektroden der Vielzahl von Elektroden 305_n des ersten Elektrodenpaars als unmittelbar benachbarte Elektrode teilen. Zwischen der einen Elektrode der Vielzahl von Elektroden 305_n des zweites Elektrodenpaars und der distalen Elektrode 301 wird nun die Spannung gemessen und über das Ohm'sche Gesetz die Gewebeimpedanz ermittelt.

Die Signalgenerator-Auswerteeinheit ist dazu eingerichtet, beispielsweise mittels einer Multiplexer-Schaltung, nacheinander zwischen allen möglichen übernächst benachbarten Elektroden den elektrischen Strom 314_m anzulegen, sodass stets eine Elektrode der Vielzahl von Elektroden übersprungen wird, zu der die Spannung gemessen wird.

Im vorliegenden Fall ist die Vielzahl von Elektroden 305_n beispielhaft als 15 Elektroden ausgebildet. Mittels der zuvor beschriebenen Ansteuerung können, wenn nacheinander an alle möglichen übernächst benachbarten Elektroden der elektrische Strom angelegt wurde und über die übersprungene Elektrode die elektrische Spannung gemessen wurde, 15 lokale Gewebeimpedanzen aus den Messwerten ermittelt werden.

Figur 8 zeigt die Vorrichtung für die "One-Shot"-Konfiguration 300 zur Ansteuerung der Elektroden und zur Abgabe von IRE-Pulsen. Die Vorrichtung weist eine distale Elektrode 301, eine zusätzliche Elektrode 303, eine proximale Elektrode 306 und eine Gegenelektrode 320 auf. Die zwischen der zusätzlichen Elektrode 303 und der proximalen Elektrode 306 angeordnete Membran 304 ist in einem zweiten Formzustand; die Membran ist expandiert. Auf der Membran 304 ist eine Vielzahl von Elektroden 305_n angeordnet. Hierbei ist das erste elektrische Signal als elektrisches Spannungssignal (321_n, 322_p, 323_n) ausgebildet.

Die hier nicht dargestellte Signalgenerator-Auswerteeinheit steuert ein erstes Elektrodenpaar an, über welches die Ablation durchgeführt wird. In der schematischen Darstellung sind drei (1)(2)(3) erste Elektrodenpaare dargestellt, über die die Ablation durchgeführt wird: (1) zwei Elektroden der Vielzahl von Elektroden 305_n, (2) eine der Vielzahl von Elektroden und die distale Elektrode 301, sowie (3) eine der Vielzahl von Elektroden und die Gegenelektrode 320.

Steuert die Signalgenerator-Auswerteeinheit das dritte (3) erste Elektrodenpaar an, erfolgt die Abgabe der IRE-Pulse 323_n über jenes Elektrodenpaar und das resultierende elektrische Feld entsteht dabei zwischen jeweils einer der Vielzahl von Elektroden 305_n und der Gegenelektrode 320. Mittels der Signalgenerator-Auswerteeinheit, die beispielsweise hierzu eine Multiplexer-Schaltung aufweist, werden sukzessive alle weiteren Elektroden der Vielzahl von Elektroden 305_n angesteuert, sodass jede der Vielzahl von Elektroden 305_n nach Ablauf der Ablationsprozedur zumindest einmal aktiv war und die IRE-Pulse 323_n jeweils zur Gegenelektrode hin abgegeben werden konnten.

Steuert die Signalgenerator-Auswerteeinheit das zweite (2) erste Elektrodenpaar an, ergibt sich der Ablauf der Ablationsprozedur wie im vorherigen Absatz, jedoch werden die IRE-Pulse 321_n jeweils zur distalen Elektrode 301 hin abgegeben.

Steuert die Signalgenerator-Auswerteeinheit das erste (1) erste Elektrodenpaar an, erfolgt die Abgabe der IRE-Pulse 322_p über jenes Elektrodenpaar und das resultierende elektrische Feld entsteht dabei zwischen zwei unmittelbar benachbarten Elektroden der Vielzahl von Elektroden 305_n.

Mittels der Signalgenerator-Auswerteeinheit, die hierzu beispielsweise eine Multiplexer-Schaltung aufweist, werden sukzessive alle Elektroden paarweise durchgeschaltet, bis alle Elektroden zumindest einmal aktiv waren.

Für ein iteratives Durchschalten der Elektroden des dritten ersten Elektrodenpaars gilt, dass ein erstes Elektrodenpaar aus einer aktuellen Iteration des Ansteuer-/Schaltungsvorgangs mit einem ersten Elektrodenpaar der vorhergehenden Iteration eine Elektrode der Vielzahl von Elektroden gemein haben. Alle drei Elektrodenpaare erlauben aufgrund des Multiplexings die Realisierung eines kreisförmigen Läsions-Musters umlaufend zur Pulmonalvene. Die Ansteuerung der Elektrodenpaare (1) und (2) stellt jeweils eine monopolare Ablations-Konfiguration dar. Die Ablation über das dritte (3) erste Elektrodenpaar stellt eine bipolare Ablations-Konfiguration dar.

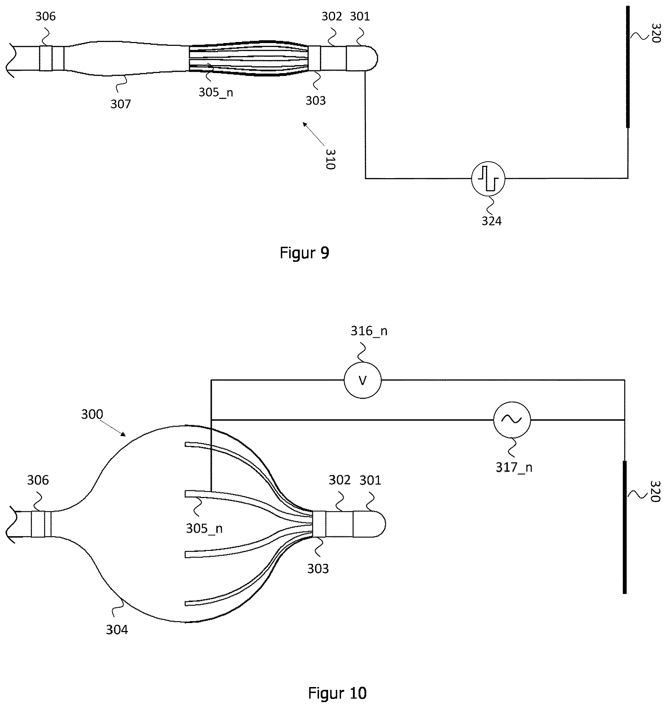

Figur 9 zeigt die Vorrichtung in der fokalen Konfiguration 310 zur Ansteuerung eines Elektrodenpaares zur IRE-Pulsabgabe. Auch hier kommt eine KörperoberflächenElektrode 320 zum Einsatz, die als Gegenpol zu der distalen Elektrode 301 dient. Somit können die IRE-Pulse 324 abgegeben werden. Diese Ablation stellt eine monopolare Ablations-Konfiguration dar.

Figur 10 zeigt schematisch die Messung einer globalen Impedanz der Vorrichtung in einem expandierten Zustand der Membran zur Bestimmung der globalen Gewebeimpedanz des Gewebes. Die Vorrichtung weist eine distale Elektrode 301, eine zusätzliche Elektrode 303, eine proximale Elektrode 306 und eine Gegenelektrode 320 auf. Die zwischen der zusätzlichen Elektrode 303 und der proximalen Elektrode 306 angeordnete Membran 304 ist in einem zweiten Formzustand; die Membran 304 ist expandiert. Auf der Membran 304 ist eine Vielzahl von Elektroden 305_n angeordnet. Hierbei ist das erste elektrische Signal als elektrisches Stromsignal 317_n ausgebildet. Das zweite elektrische Signal ist als elektrisches Spannungssignal 316_n ausgebildet. Die hier nicht dargestellte Signalgenerator-Auswerteeinheit steuert ein erstes Elektrodenpaaren an. Das erste Elektrodenpaar ist aus einer Elektrode der Vielzahl von Elektroden 305_n und der Gegenelektroden 320 gebildet. Über das erste Elektrodenpaar wird der elektrische Strom in das Gewebe gesendet und die elektrische Spannung gemessen. Die nicht dargestellte Signalgenerator-Auswerteeinheit bestimmt aus dem elektrischen Strom und der elektrischen Spannung über das ohmsche Gesetz eine globale Gewebeimpedanz.

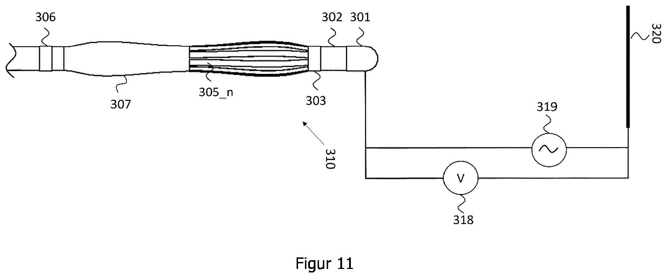

Figur 11 zeigt schematisch die Messung einer globalen Impedanz der Vorrichtung in einem kollabierten Zustand der Membran. Die Vorrichtung weist eine distale Elektrode 301, eine zusätzliche Elektrode 303, eine proximale Elektrode 306 und eine Gegenelektrode 320 auf. Die zwischen der zusätzlichen Elektrode 303 und der proximalen Elektrode 306 angeordnete Membran 304 ist in einem ersten Formzustand; die Membran 304 ist kollabiert. Auf der Membran 304 ist eine Vielzahl von Elektroden 305_n angeordnet. Hierbei ist das erste elektrische Signal als elektrisches Stromsignal 319 ausgebildet. Das zweite elektrische Signal ist als elektrisches Spannungssignal 318 ausgebildet. Die hier nicht dargestellte Signalgenerator-Auswerteeinheit steuert ein erstes Elektrodenpaaren an. Das erste Elektrodenpaar ist aus der distalen Elektrode 301 und der Gegenelektroden 320 gebildet. Über das erste Elektrodenpaar wird der elektrische Strom in das Gewebe gesendet und die elektrische Spannung gemessen. Die nicht dargestellte Signalgenerator-Auswerteeinheit bestimmt aus dem elektrischen Strom und der elektrischen Spannung über das ohmsche Gesetz eine globale Gewebeimpedanz.

## Patentansprüche

1. Vorrichtung zur irreversiblen Elektroporation eines Gewebes eines Patienten aufweisend:
- einen Katheter aufweisend
- ein proximales Ende und eine an einem distalen Ende des Katheters angeordnete distale Elektrode;
- eine zwischen dem distalen Ende und dem proximalen Ende angeordnete Membran, die dazu eingerichtet ist, einen ersten und einen zweiten Formzustand einzunehmen;
- eine Vielzahl von Elektroden, die auf der Membran angeordnet sind;
- eine mit dem proximalen Ende des Katheters verbundene Signalgenerator-Auswerteeinheit, die dazu eingerichtet, eine Gewebeimpedanzbestimmung und/oder eine Ablation durchzuführen.

2. Vorrichtung nach Anspruch 1, wobei die Vorrichtung aufweist:
- eine an dem proximalen Ende des Katheters angeordnete proximale Elektrode; und/oder
- zumindest eine zusätzliche Elektrode, die zwischen der Membran und der distalen Elektroden angeordnet ist.

3. Vorrichtung nach Anspruch 1 oder 2, wobei die Signalgenerator-Auswerteeinheit dazu eingerichtet ist, über eine Elektrodenkonstellation ein erstes elektrisches Signal in das Gewebe abzugeben, oder ein erstes elektrisches Signal in das Gewebe abzugeben und ein zweites elektrisches Signal über die Elektrodenkonstellation aus dem Gewebe zu empfangen.

4. Vorrichtung nach Anspruch 3, wobei die Elektrodenkonstellation aufweist:
- die proximale und distale Elektrode als erstes Elektrodenpaar und die zusätzliche Elektrode und die distale Elektrode als zweites Elektrodenpaar, insbesondere in dem ersten Formzustand; oder
- die proximale und distale Elektrode als erstes Elektrodenpaar und eine der Vielzahl von Elektroden und die zusätzliche Elektrode, insbesondere in dem zweiten Formzustand, als zweites Elektrodenpaar; oder
- zwei zueinander übernächst benachbarte Elektroden der Vielzahl von Elektroden als erstes Elektrodenpaar und die distale Elektrode und eine, insbesondere zwischen den zwei übernächst benachbarten Elektroden gelegene, weitere Elektrode der Vielzahl von Elektroden, insbesondere in dem zweiten Formzustand, als zweites Elektrodenpaar.

5. Vorrichtung nach einem der Ansprüche 1 bis 4, wobei in einer ersten Betriebsphase:
- das erste elektrische Signal als elektrisches Stromsignal und das zweite elektrische Signal als elektrisches Spannungssignal ausgebildet ist; und
- die Signalgenerator-Auswerteeinheit dazu eingerichtet ist, aus dem in das Gewebe geleiteten elektrischen Stromsignal und dem aus dem Gewebe empfangenen elektrischen Spannungssignal zumindest eine, insbesondere lokale, Gewebeimpedanz zu bestimmen.

6. Vorrichtung nach Anspruch 5, wobei die Signalgenerator-Auswerteeinheit dazu eingerichtet ist, mittels, insbesondere (zeitlich) nacheinander gewählten/geschalteten, zumindest zwei Elektrodenkonstellationen zumindest zwei, insbesondere lokale, Gewebeimpedanzen zu bestimmen.

7. Vorrichtung nach einem der Ansprüche 2 bis 6, wobei die Signalgenerator-Auswerteinheit dazu eingerichtet ist, innerhalb einer Elektrodenkonstellation, insbesondere zeitlich nacheinander, zumindest ein anderes erstes und/oder zweites Elektrodenpaar anzusteuern und für die Elektrodenkonstellation zumindest zwei, insbesondere lokale, Gewebeimpedanzen zu bestimmen.

8. Vorrichtung nach einem der Ansprüche 2 bis 7, wobei die Signalgenerator-Auswerteeinheit dazu eingerichtet ist, für
- die proximale und distale Elektrode als erstes Elektrodenpaar und eine der Vielzahl von Elektroden und die zusätzliche Elektrode, insbesondere in dem zweiten Formzustand, als zweites Elektrodenpaar,
das zweite Elektrodenpaar zumindest einmalig aus zumindest einer weiteren Elektrode der Vielzahl von Elektroden und der zusätzlichen Elektroden, insbesondere in dem zweiten Formzustand, anzusteuern und zumindest eine weitere, insbesondere lokale, Gewebeimpedanz zu bestimmen, und/oder die Signalgenerator-Auswerteeinheit dazu eingerichtet ist, für
- zwei zueinander übernächst benachbarte Elektroden der Vielzahl von Elektroden als erstes Elektrodenpaar und die distale Elektrode und eine, insbesondere zwischen den zwei übernächst benachbarten Elektroden gelegene, weitere Elektrode der Vielzahl von Elektroden, insbesondere in dem zweiten Formzustand, als zweites Elektrodenpaar,
das erste Elektrodenpaar zumindest einmalig aus zwei weiteren übernächst benachbarten Elektrode der Vielzahl von Elektroden, insbesondere in dem zweiten Formzustand, und die distale Elektrode und eine, insbesondere zwischen den zwei weiteren übernächst benachbarten Elektroden gelegene, weitere Elektrode der Vielzahl von Elektroden, insbesondere in dem zweiten Formzustand, als zweites Elektrodenpaar anzusteuern und zumindest eine weitere, insbesondere lokale, Gewebeimpedanz zu bestimmen.

9. Die Vorrichtung nach einem der Ansprüche 1 bis 8, wobei in einer zweiten Betriebsphase:
- das elektrische Signal als elektrisches Spannungssignal ausgebildet ist; und
- die Signalgenerator-Auswerteeinheit dazu eingerichtet ist, das elektrische Spannungssignal gemäß einem zu wählenden Burst-Signal-Protokoll zu generieren und über ein erstes Elektrodenpaar in das Gewebe zu leiten; wobei
- die distale Elektrode und eine der Vielzahl von Elektroden, insbesondere in einem zweiten Formzustand, als erstes Elektrodenpaar ausgebildet ist, oder
- zwei, insbesondere benachbarte, Elektroden der Vielzahl von Elektroden, insbesondere in einem zweiten Formzustand, als erstes Elektrodenpaar ausgebildet ist.

10. Vorrichtung nach einem der Ansprüche 1 bis 9, wobei die Vorrichtung weiter eine mit der Signalgenerator-Auswerteeinheit verbundene Gegenelektrode, insbesondere Körperflächen-Gegenelektrode, aufweist, wobei:
- eine der Vielzahl von Elektroden und die Gegenelektrode, insbesondere in dem zweiten Formzustand, als erstes Elektrodenpaar ausgebildet ist; und/oder
- die distale Elektrode und die Gegenelektrode, insbesondere in dem ersten Formzustand, als erstes Elektrodenpaar ausgebildet ist, und/oder in einer ersten Betriebsphase:
- das erste elektrische Signal als elektrisches Stromsignal und das zweite elektrische Signal als elektrisches Spannungssignal ausgebildet ist; und
- die Signalgenerator-Auswerteeinheit dazu eingerichtet ist, aus dem, insbesondere über das erste Elektrodenpaar, in das Gewebe geleiteten elektrischen Stromsignal und dem, insbesondere über das erste Elektrodenpaar, aus dem Gewebe empfangenen elektrischen Spannungssignal zumindest eine, insbesondere globale, Gewebeimpedanz zu bestimmen.

11. Vorrichtung nach Anspruch 9 oder 10, wobei die Signalgenerator-Auswerteeinheit dazu eingerichtet ist, innerhalb eines Elektrodenpaars, insbesondere zeitlich nacheinander, zu zumindest einer weiteren Elektrode der Vielzahl von Elektroden zu schalten.

12. Vorrichtung nach einem der Ansprüche 1 bis 11, wobei die Elektroden, insbesondere die Vielzahl von Elektroden, die proximale, die distale und die zusätzliche Elektrode, mittels untereinander und zur unmittelbaren Umgebung isolierten, insbesondere außen auf dem Katheter angeordneten, elektrischen Leitungen über das proximale Ende des Katheters mit der Signalgenerator-Auswerteeinheit verbunden sind.

13. Verfahren zur irreversiblen Elektroporation eines Gewebes eines Patienten umfassend die Schritte:
- Bereitstellen eines Katheters aufweisend
- ein proximales Ende und eine an einem distalen Ende des Katheters angeordnete distale Elektrode;
- eine zwischen dem distalen Ende und dem proximalen Ende angeordnete Membran, die dazu eingerichtet ist, einen ersten und einen zweiten Formzustand einzunehmen;
- eine Vielzahl von Elektroden, die auf der Membran angeordnet sind;
- Bereitstellen einer mit dem proximalen Ende des Katheters verbundenen Signalgenerator-Auswerteeinheit; und
- Durchführen einer Gewebeimpedanzbestimmung oder Durchführen einer Ablation, insbesondere Durchführen einer Gewebeimpedanzbestimmung und anschließendes Durchführen einer Ablation.
